# EUROPEAN PATENT APPLICATION

(11) **EP 2 942 387 A2**
(43) Date of publication of application: **11.11.2015**
(21) Application number: 15165521.4
(22) Date of filing: 28.04.2015
(51) Int. Cl.: C12M 1/00, C12M 1/12, C12M 1/34

(54) **SYSTEM AND METHOD FOR USING A PULSE FLOW CIRCULATION FOR ALGAE CULTIVATION**

(30) Priority: 06.05.2014 US 201414270800
(71) Applicant: General Atomics, San Diego, California 92121-1194 (US)
(72) Inventor: HAZLEBECK, David A., El Cajon, CA California 92020 (US); ZHANG, Jiping, San Diego, CA California 92129 (US); DOWNEY, Kevin W., San Diego, CA California 92128 (US); WU, Xiaoxi, Encinitas, CA California 92024 (US)
(74) Representative: Gates, Marie Christina Esther

(57) **Abstract**

A device for generating a pulsed flow in a channel containing a circulating algal culture can include a plate that is pivotably mounted on the channel and an activator. A pulsed flow is generated in the channel by first positioning the plate to impede the flow of circulating algal culture and then rotating the plate to a submerged position. The pulsed flow can be employed to counteract the negative effects of bio-fouling on algae cultivation equipment. In another arrangement, a device for generating a pulsed flow in a sloped raceway that is in fluid communication with a sump can include a gate. In different embodiments, the gate can operate as a so-called "pinch gate" or as a so-called "overflow gate." In another aspect, a variable rate pump, such as a centrifugal pump, a screw pump or an airlift pump, is described for establishing a pulsed flow in a channel.

## Description

This application is a continuation-in-part (CIP) application of Application Serial No. 13/973,638 filed August 22, 2013, which is currently pending and is a divisional of Application Serial No. 13/189,737, filed July 25, 2011, which issued as U.S. Patent No. 8,541,225 on September 24, 2013. The contents of Application Serial No. 13/973,638 and Patent No. 8,541,225 are incorporated herein by reference.

### FIELD OF THE INVENTION

The present invention pertains generally to systems and methods for growing algae. More particularly, the present invention pertains to the use of a system that can continuously grow algae in a more efficient manner by minimizing complications caused by biofouling. The present invention is particularly, but not exclusively, useful as a system for increasing the productivity of algae growth systems by using a pulse flow to periodically stir and rinse the algae cultivation apparatus and to increase the available surface area of the culture.

### BACKGROUND OF THE INVENTION

As worldwide petroleum deposits decrease, there is rising concern over petroleum shortages and the costs that are associated with the production of carbon-based fuel sources. As a result, alternatives to products that are currently processed from petroleum are being investigated. In this effort, biofuel has been identified as a possible alternative to petroleum-based fuels. In general, a biodiesel is a fuel comprised of mono-alkyl esters of long chain fatty acids derived from plant oils or animal fats. In industrial practice, biodiesel is created when plant oils or animal fats are reacted with an alcohol, such as methanol.

Apart from using animal fats, the creation of biofuels from plant oils has gained wide attention in recent years. The process of creating biofuel from plant oils, of course, necessarily begins by growing and harvesting plants such as algae cells. In particular, algae is known to be one of the most efficient plants for converting solar energy into cell growth, so it is of particular interest as a biofuel source.

In an algae cultivation system, the algae cells are typically grown in a cultivation apparatus as part of a liquid medium that is exposed to sunlight to promote photosynthetic growth. Further, the algae cell growth process normally requires the liquid medium to be continuously circulated through the system to allow algae cells to ingest nutrients. Three of the most prevalent algae cultivation apparatuses in use today which meet these requirements are: (1) a photobioreactor, (2) a cultivation pond with a circulation device, and (3) a cultivation pond without a circulation device. Despite having numerous advantages when growing algae, these apparatuses have significant disadvantages, many of which involve biofouling. With biofouling, algae cells tend to adhere to or accumulate on various surfaces. In particular, the algae cells adhere to a light transmitting cover and to the bottom and walls of the apparatus. Importantly, biofouling can significantly decrease the productivity of an algae cultivation system. In detail, if biofouling occurs because algae cells adhere to the light transmitting cover, photosynthesis is disrupted as less light reaches algae cells. In addition, when algae cells remain stationary on a surface, several problems arise: (1) algae cells may die and provide a food source for contaminants like protozoa; (2) algae cells settled too deep below the surface of the water will not receive enough light; and (3) algae cells will not move enough to ingest nutrients floating in the algal culture. All of these problems cause significant disruptions to an effective algae cultivation system.

Various efforts have been made to continuously circulate algal culture. Yet, biofouling still causes significant problems to algae cultivation systems. For one, system efficiency is hindered as algae cultivation systems must be drained and cleaned often to remove the algae cells that have adhered to various surfaces. These interruptions can be minimized by using a device or method that forcefully removes algae cells from surfaces and also serves as an impetus to circulate algae cells in the system.

As described further herein, a pulsed flow can be generated and used to provide high localized and periodic fluid velocities within a circulating algal culture that can aid in moving dust and other pond sediments along the length of a pond to a central collection point where they can be easily removed (e.g., a sump). This action can result in a cleaner pond system. In addition to cleaning, waves generated by pulse flow can increase the equivalent surface area of the pond, leading to a higher growth for a given pond area. Together with the rougher fluid surface produced by the pulses, less light is reflected, and more light energy is absorbed. The wavy surface of the pulsed flow system can also create a better angle for incident light. In some instances, as much as 40% more surface area can be created by a wave created by a pulse flow resulting in an overall algae growth rate that is enhanced by 20% or more. Also, for tubular reactors, the sudden increase in flow rate that results from a pulsed flow can help transport stationary gas bubbles to an open space for release. The removal of gas bubbles can be beneficial since the bubbles often have a high oxygen concentration that can inhibit photosynthesis.

In light of the above, it is an object of the present invention to provide a system and method for growing algae for biofuel production which minimizes the effects of biofouling. Another object of the present invention is to provide a system and method for growing algae that uses a pulse flow to increase the efficiency of the system. Still another object of the present invention is to provide devices for generating pulsed flows in algal cultures circulating in channels and conduits. Yet another object of the present invention is to provide a system and method for growing algae using pulse flow circulation that is simple to implement, easy to use, and comparatively cost effective.

### SUMMARY OF THE INVENTION

In accordance with the present invention, a system and method for using a pulse flow to circulate algae in an algae cultivation system is provided. In one embodiment, an elevated flush tank is used to create the pulse flow. When it is created, the pulse flow circulates algae cells and dislodges any algae cells clinging to various components of the algae cultivation system.

Structurally, the system of the present invention may be adapted for use with any type of algae cultivation device presently in use, or the system may be used as a stand-alone algae cultivation system. The two most common devices for cultivating algae in use today are a photobioreactor or a pond (with or without a circulation device). The present invention can be adapted for use with either type of device. For comparison purposes, the photobioreactor is a closed system that most often has a vertical configuration, while the pond is an open system and is built in a horizontal configuration onto a surface. In either case, a flush tank is provided to store a fixed amount of algal culture effluent which has been drawn from the algae cultivation device. As contemplated for the present invention, the flush tank is situated higher than the cultivation device and is connected to the cultivation device by a conduit. To account for this difference in elevation, a circulation pump is provided to move the effluent from the cultivation device into the flush tank. This pump is usually located at an access point or a drainage point of the algae cultivation device. To be more specific, because a photobioreactor is usually constructed with a vertical orientation, an access or drainage point is most often situated at the bottom of the photobioreactor. And, a cultivation pond will have a designated drainage or access point for removing liquid from the pond. In either case, a conduit is connected between the circulation pump and the flush tank. In addition, a gas exchange tank may also be included in the system to add carbon dioxide (CO₂) to a portion of the drawn effluent while, at the same time, removing oxygen (O₂). The gas exchange tank is included in the system to promote algae growth by providing CO₂ to be used as a nutrient source by algae cells. As envisioned for the present invention, the gas exchange tank receives algal effluent drawn from the algae cultivation device, enriches the effluent with CO₂, and reintroduces the CO₂-rich effluent back into the cultivation device through a return pipe. Furthermore, the gas exchange tank and the flush tank can be the same one.

Several components may be provided to govern the release of the effluent from the flush tank. For one, a timer may be connected to the flush tank to release the effluent at a predetermined time. In another embodiment, a level switch is connected to the flush tank to release the effluent once the flush tank reaches a preplanned capacity level. As envisioned for the present invention, the timer and the level switch are both included for use with the system. Alternatively, the flush tank may also be manually activated with an activation switch. With any of the activation methods, a gate valve is moved from a closed position to an open position to release the fluid from the flush tank into a transfer pipe connected to the algae cultivation device. Upon activation, a pulse flows rapidly from the flush tank into the algae cultivation device.

In operation, the system of the present invention begins by drawing a portion of algal culture from the algae cultivation device to create an effluent. The effluent is then pumped, using the circulation pump, into the flush tank via the conduit. The effluent remains in the flush tank until it is released in one of the following ways: (1) the flush valve is manually opened; (2) the timer initiates the activator to open the flush valve; or (3) a level switch initiates the flush valve to release the effluent once the effluent reaches a predetermined level in the flush tank. It should be noted that the system may use any combination of the preceding methods for releasing effluent from the flush tank. Upon release, and due primarily to the elevation difference between the cultivation device and the flush tank, the effluent will flow rapidly out of the flush tank and into the cultivation device through a transfer pipe to create a pulse flow of effluent. Due to the sudden increase in the fluid flow rate, the pulse flow will dislodge any algae cells which are attached to any surface of the cultivation device. Additionally, the pulse flow will cause turbulence in the algae cultivation device. This turbulence will cause most, if not all, of the algae cells, which have settled onto the bottom of the cultivation device, to become suspended once again in the algal culture. This movement of the algae cells will promote photosynthesis and improve access to nutrients floating in the culture. In most cases, the direction of flow for the pulse flow will be the same direction as the flow in the cultivation device. Yet, the system may also reverse the flow direction of the pulse flow to go in the opposite direction of the algal culture flow in the cultivation device.

Once the effluent is released, the flush tank is emptied, and a new pulse flow cycle, or flush cycle, can begin. A new cycle begins when the flush valve is closed and the pump draws effluent to fill the flush tank.

In another aspect, a device for generating a pulsed flow in a channel containing a circulating algal culture can include one or more plates that are positioned in the channel. For example, the channel can be a photo-bioreactor (PBR) tube, a pond channel or a sloped raceway. For this aspect, the plate has a first plate end and a second plate end with the first plate end pivotably mounted on the channel. An activator, such as a rotary actuator, is coupled with the plate for selectively rotating the plate about a pivot axis, back and forth, between a first plate position and a second plate position. In the first plate position, the second end of the plate is positioned above the algal culture and in the second plate position, the second end of the plate is submerged in the algal culture.

With the above described arrangement, a pulsed flow can be generated in the channel by first rotating the plate from the second submerged position to the first position. In the first position, the plate impedes the flow of circulating algal culture producing a height differential between algal culture on opposing sides of the plate. After a preselected time, or when a preselected height differential across the plate is achieved, the actuator can be activated to rotate the plate back to the second position (i.e. the submerged position) to allow the buildup of algal culture to flow, substantially unimpeded, along the channel. This release causes a pulsed flow (i.e. wave) in the channel. For this purpose, the activator can include a timer for initiating a pulsed flow in accordance with a predetermined schedule and/or for ensuring that the second end of the plate is positioned above the algal culture for a preselected period of time during a pulse generation sequence. Alternatively, the activator can include a level switch for selectively releasing a buildup of algal culture by rotating the plate after a preselected height differential has been established across the plate. In some cases, the plate can be less than the full width of the channel to allow a portion of the fluid flow to bypass the plate when in the first position, thereby maintaining a prescribed minimum flow in the channel. The pulsing action of the periodically-increased flow rate produces waves that propagate the length of the channel. The waves have high points and low points that overall yield a higher surface area for the algae culture, by up to 40%, than would otherwise be available with a non-pulsed channel. The increased surface area provides greater culture productivity since productivity is a function of surface area.

In another aspect, a device for generating a pulsed flow in a sloped raceway that is in fluid communication with a sump can include a gate that is formed as a planar plate. For this aspect, the gate has a first end and a second end and defines a plane. The gate is interposed between the sump and the sloped raceway and is mounted on the sump-raceway assembly to allow a back and forth movement of the gate within the defined plane. To control movement of the gate, the gate is coupled to an activator, such as a linear actuator. With this cooperative structural interaction, the gate can be selectively moved within the defined plane from a first position to a second position. In the first gate position, a flow rate *f₁* is established from the sump to the raceway and in the second gate position a flow rate *f₂* is established from the sump to the raceway, with f₂ > f₁. To generate a pulsed flow in the conduit, the gate is moved from the first position to the second position. For this aspect, the activator can include a timer for initiating plate movements to established pulsed flows in accordance with a predetermined schedule. Alternatively, or in addition to the timer, the activator can include a level switch for measuring a height of algal culture in the sump and for initiating plate movements when the algal culture reaches a predetermined height.

In a first embodiment of this aspect, the gate can operate as a so-called "pinch gate." For this embodiment, the sump is attached to the raceway at an edge that is submerged in algal culture. To create a pulse, the gate is initially positioned to locate the first gate end at a distance *d₁* from the submerged edge, allowing algal culture to accumulate behind the gate in the sump. When a pulse is needed, the gate is opened by moving it to the second gate position where the first gate end is at a distance *d₂* from the submerged edge, with d₂ > d₁. Note, for this embodiment, the gate can be slightly open in the first position d₁ > 0 to allow the algal culture to circulate, or the gate can be temporarily closed d₁ = 0.

In another embodiment of this aspect, the gate can operate as a so-called "overflow gate." For this embodiment, the gate is moved to position the second gate end above the algal culture to allow algal culture to accumulate behind the gate. When a pulse is needed, the gate is opened by moving it to the second gate position in which the second end of the gate is submerged in the algal culture and algal culture flows over the second end of the gate to establish the pulsed flow.

In yet another aspect, a device for generating a pulsed flow in a channel containing a circulating algal culture can include a pump and an activator. For this aspect, the pump is coupled to the actuator for selectively increasing the flow rate in the channel from a first flow rate *f₁* established for circulating the algal culture through the channel to a second flow rate *f₂* with f₂ > f₁ to generate a pulsed flow in the channel. For example, the pump can be a variable rate pump such as a centrifugal pump, a screw pump or an airlift pump. In one setup, flow is increased to establish a pulsed flow by increasing the flow rate such that the ratio of flow rates (f₂ : f₁) is in a range between about 2:1 and about 10:1. In one embodiment of this aspect, the pump can include one or more lifting buckets and the actuator can include a motorized track for moving the bucket(s) from a channel fill location where a bucket is filled to a channel dump location where a bucket is emptied. For this aspect, the activator can include a timer for initiating pump rate changes in accordance with a predetermined schedule and/or a level switch for controlling pump operation.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Fig. 1 is a schematic diagram of the layout of the system for the present invention when used in conjunction with a photobioreactor (PBR);
Fig. 2 is a schematic diagram of the layout of the system for the present invention when used in conjunction with a raceway pond with a circulation device;
Fig. 3 is an elevation view of the system of the present invention when used in conjunction with a sloped pond without a circulation device;
Fig. 4 is a detail of one section of the system of the present invention when used in conjunction with a sloped pond;
Fig. 5 is a top view of a channel for circulating an algal culture and a device for generating a pulsed flow in the channel;
Fig. 6 is a sectional view of the channel as seen along line 6-6 in Fig. 5;
Fig. 6A is a sectional view as in Fig. 6 of another embodiment in which a plate can be rotated very quickly to pinch algal culture between the plate and channel floor to generate a pulsed flow;
Fig. 6B is a sectional view as in Fig. 6 of another embodiment in which a plate can be rotated very quickly to push algal culture and generate a pulsed flow;
Fig. 7 is a sectional view, as in Fig. 6, showing another embodiment of a device for generating a pulsed flow in a sloped raceway that is in fluid communication with a sump, the device having a "pinch gate" and shown with the gate in the closed position to allow algal culture to circulate and accumulate in the sump;
Fig. 8 is a sectional view of the Fig. 7 embodiment shown with the gate in the open position to generate a pulsed flow in a sloped raceway;
Fig. 9 is a sectional view, as in Fig. 6, showing another embodiment of a device for generating a pulsed flow in a sloped raceway that is in fluid communication with a sump, the device having an "overflow gate" and shown with the gate in the closed position to allow algal culture to circulate and accumulate in the sump;
Fig. 10 is a sectional view of the Fig. 9 embodiment shown with the gate in the open position to generate a pulsed flow in a sloped raceway;
Fig. 11 is a top view of a channel for circulating an algal culture and a device having a variable rate pump for generating a pulsed flow in the channel;
Fig. 12 is a top view of a channel for circulating an algal culture and a device for generating a pulsed flow in the channel having a plurality of lifting buckets and a motorized track for moving the buckets from a channel fill location to a channel dump location;
Fig. 13 is a sectional view as in Fig. 6 of another embodiment in which a plate and trigger cooperate to generate a pulsed flow, shown with the plate held in a closed position by the trigger;
Fig. 14 is a sectional view of the embodiment shown in Fig. 13, shown with the plate in an open position; and
Fig. 15 is a sectional view of the embodiment shown in Fig. 13, shown after the plate has returned to the closed position under the influence of gravity.

### DESCRIPTION OF THE PREFERRED EMBODIMENTS

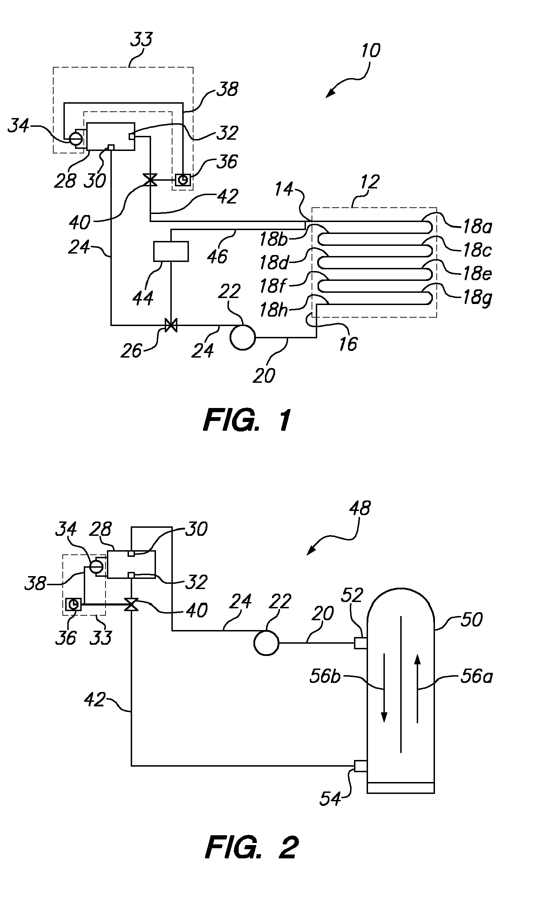

Initially referring to Fig. 1, a system of the present invention is shown and is generally designated 10. In Fig. 1, the system 10 is shown when used in conjunction with a photobioreactor 12 having an inlet 14 for introducing algal culture into the photobioreactor 12 and an outlet 16 for removing algal culture from the photobioreactor 12 as needed. In addition, the photobioreactor 12 is built with a plurality of flow channels 18a-h with algal culture traveling through the photobioreactor starting at flow channel 18a. As shown, the outlet 16 of the photobioreactor 12 is connected to a transfer pipe 20 through which circulation pump 22 can draw a portion of growing algal culture out of the photobioreactor 12. Furthermore, the circulation pump 22 is connected to a conduit 24 which includes a diverting valve 26 that is used to redirect algal culture from the conduit 24. The diverting valve 26 remains in a closed position when algal culture is being pumped to a flush tank 28, which is constructed with an inlet 30 and an outlet 32. As required for the present invention, the flush tank 28 stores drawn algal culture until released. In order to release the drawn algal culture, an activator 33 is provided. In Fig. 1, it can be seen that the activator 33 comprises a level switch 34 or a timer 36 electrically connected by a signal wire 38. Once initiated, the activator 33 will open gate valve 40 to release the drawn algal culture into a transfer pipe 42 connected between the flush tank 28 and the inlet 14 of the photobioreactor 12.

In order to promote algae growth, a gas exchange tank 44 is provided to provide CO₂ to algal culture in the photobioreactor 12. To do this, the diverting valve 26 is opened and effluent is pumped out of the photobioreactor 12. This effluent is diverted to the gas exchange tank 44 where CO₂ is added to the effluent and O₂ is removed. Once this gas exchange process is completed, the effluent from the gas exchange tank 44 will travel through a return pipe 46 to the photobioreactor 12 to provide nutrients to the growing algal culture. As envisioned for the present invention, diverting effluent to the gas exchange tank 44 can be accomplished independent of filling the flush tank 28 or at the same time as the flush tank 28 is filled, or the system can be arranged so that the flush tank is used for gas exchange purposes as well, and therefore the independent gas exchange tank 44 and the diverting valve 26 can be eliminated.

Now referring to Fig. 2, a system 48 for the present invention, when used in conjunction with a raceway pond 50 containing algal culture, is shown. In this embodiment, many of the components are identical to the embodiment shown in Fig. 1. In the system 48, a circulation pump 22 removes an effluent of the algal culture out of the raceway pond 50 through a drainage point 52 into the transfer pipe 20. The effluent then moves through the circulation pump 22 and into the conduit 24 connected to the inlet 30 of the flush tank 28. Like the system 10 in Fig. 1, the activator 33, comprising a level switch 34 or a timer 36, controls the opening of the gate valve 40 which allows the effluent to be released from the flush tank 28 through its outlet 32. Once released, the effluent travels rapidly through transfer pipe 42 and into the raceway pond 50 through an inlet 54 to create a pulse flow. In system 48, the pulse flow will generate turbulence to force algae settled on the bottom of the raceway pond 50 off of the bottom and back into the culture which is flowing in the direction of arrows 56a-b. In addition, if the raceway pond 50 has a cover (not shown), algae attached to the cover will be dislodged and flow around the raceway pond 50.

Unlike the system 10 in Fig. 1, a gas exchange tank 44 is not required for the system 48 shown in Fig. 2 because the raceway pond 50 is an open system, unlike the photbioreactor 12 which is a closed system. Thus, carbon-based nutrients are easier to add directly to an open system like the raceway pond 50. But, a gas exchange tank 44 and associated equipment (i.e. diverting valve 26 and return pipe 46) can be added to the system 48, if desired, in a similar configuration as shown in Fig. 1. Alternatively, the gas exchange can take place in the flush tank 28.

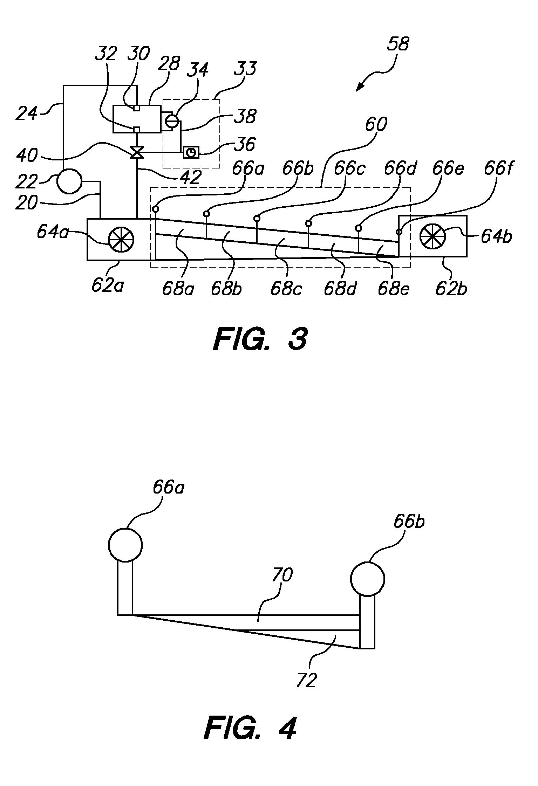

Now referring to Fig. 3, a system 58 is shown when adapted for use with a sloped pond 60. As can be seen, the circulation pump 22, the flush tank 28, level switch 34, timer 36, and gate valve 40 are substantially identical and operate in the same manner as systems 10 and 48 in Fig. 1 and Fig. 2 respectively. Importantly, the pulse flow is created in the same manner with effluent being released from the flush tank 28. Instead of entering a photobioreactor 12 or a raceway pond 50, the pulse flow enters a sump 62a before reaching the sloped pond 60. When a sloped pond 60 is used for algae cultivation, a main pump 64a, housed within the sump 62a, is provided to initiate fluid flow. As shown in Fig. 3, the sloped pond 60 is constructed with a plurality of flush gates 66a-f, with the area between consecutive flush gates 66a-f being referred to as a segment 68a-e. At the end of the last segment 68e, sump 62b, housing another main pump 64b, leads to another sloped pond (not shown). It should be noted that the system 58 may also be constructed with flush gate 66a only. With only flush gate 66a, the pulse flow will travel through the entire length of the sloped pond 60 when released from the flush tank 28. When using a plurality of flush gates 66a-f, the pulse flow can be controlled as all of the flush gates 66a-f can be opened simultaneously (in the same manner as when only flush gate 66a is used), or the flush gates 66a-f can be opened one at a time. If one flush gate is opened at a time, the pulse flow will travel through one segment 68a-e at a time.

Still referring to Fig. 3, an alternate embodiment may also be described. In this alternate embodiment, the flush tank 28 is replaced by the sump 62 and houses pump 64a. In this configuration, the circulation pump 22 can be eliminated because pump 64a is configured to produce the pulse flow. With the height of the flush tank 28 eliminated, pump 64a will be constructed to compensate for the lack of gravity flow by having the ability to produce the high fluid flow rate required to produce the pulse flow. Without a flush tank 28, the level switch 34 or timer 36 are incorporated with the sump 62 and main pump 64a. In this arrangement, the main pump 64a is configured to create a pulse flow, and instead of a gate valve 40, the flush gate 66a serves to release the effluent from the sump 62. The level switch 34 or timer 36 can be set to open the flush gates 66a-f in any sequence.

Referring now to Fig. 4, a detail of a segment between two flush gates 66a-b is shown. In Fig. 4, the concept of light and dark cycles is illustrated. In more detail, when flush gates 66a-b are in a closed position, a portion of effluent remains between the flush gates 66a-b. As shown, the effluent between the gates 66a-b will generally settle in two layers or zones, a light zone 70 and a dark zone 72. Algae cells in the effluent which settle in the light zone 70 will have an increased exposure to the light necessary for photosynthesis. At the same time, the algae cells which settle in the effluent in the dark zone 72 are not exposed to enough light for photosynthesis to occur. By using the pulse flow to create turbulence to stir up, or mix, the algal culture, nearly all of the algae cells will be exposed to a suitable amount of light for photosynthesis during one or more flush cycles.

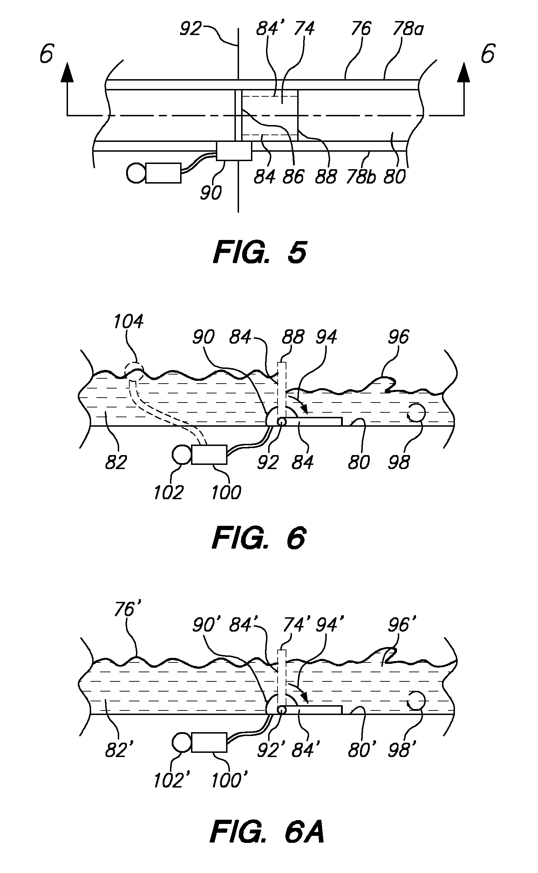

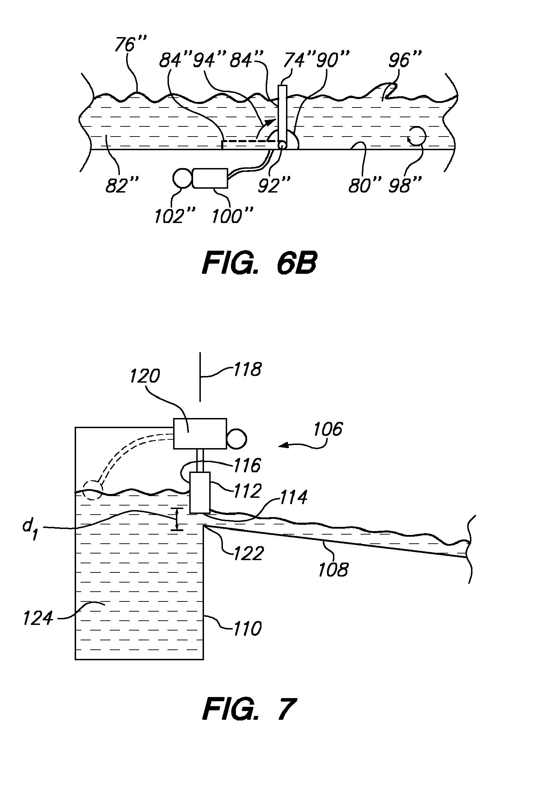

Figs. 5 and 6 show a device 74 for generating a pulsed flow in a channel 76 having channel walls 78a,b and channel floor 80 and containing a circulating algal culture 82. As shown, the device 74 includes a plate 84 that is positioned in the channel 76. Although Fig. 5 shows the plate 84 as being the full width of the channel 76, it is to be appreciated that the plate 84 can be less than the full width of the channel 76. For example, the width of the plate 84 can be in a range from about 1/3 of full channel width up to full channel width. For the device 74, the channel 76 can be a photo-bioreactor (PBR) tube, a pond channel or a sloped raceway, as described above. For example, the tubular PBRs can be hard-wall, fence-type PBRs (as with the Biofence) or soft-wall PBRs (like the submersed flexible PBRs made by Solix) or hanging tubular PBRs (as produced by Valcent). Figs. 5 and 6 show that the plate 84 has a first plate end 86 and a second plate end 88 with the first plate end 86 pivotably mounted on the channel 76. The device 74 also includes an activator 90, such as a rotary actuator, (or a linear actuator assembly [not shown]), that is coupled with the plate 84 for selectively rotating the plate 84 about a pivot axis 92. Specifically, the plate 84 can be rotated, back and forth, between a first plate position (shown in dotted lines in Fig. 6) and a second plate position (shown in Fig. 5 and in solid lines in Fig. 6). As shown, in the first plate position, the second end 88 of the plate 84 is positioned above the algal culture 82 and in the second plate position, the second end 88 of the plate 84 is submerged in the algal culture 82.

In operation, a pulsed flow can be generated in the channel 76 by first rotating the plate 84 from the submerged position to the first position (shown in dotted lines in Fig. 6). As shown, in the first position, the plate 84 impedes the flow of circulating algal culture 82 producing a height differential between algal culture 82 on opposing sides of the plate 84. After a preselected time, or when a preselected height differential across the plate 84 is achieved, the activator 90 can be activated to rotate the plate 84 to the submerged position (see arrow 94) to allow the buildup of algal culture 82 behind the plate 84 to flow, substantially unimpeded, along the channel 76. This release causes a pulsed flow (illustrated as wave 96) and can generate turbulence (illustrated by eddy flow arrow 98) in the channel 76.

Fig. 6 also shows that the activator 90 can include controller 100 and a timer 102 for initiating a pulsed flow in accordance with a predetermined schedule and/or for ensuring that the second end 88 of the plate 84 is positioned above the algal culture 82 for a preselected period of time during a pulse generation sequence. Alternatively, as shown, the activator 90 can include a level switch 104 (shown in dotted lines as optional) for selectively releasing a buildup of algal culture 82 by rotating the plate 84 after a preselected height differential has been established across the plate 84.

Fig. 6A shows an embodiment of a device 74' for generating a pulsed flow in a channel 76' having a channel floor 80' and containing a circulating algal culture 82'. As shown, the device 74' includes a plate 84' that is positioned in the channel 76'. The plate 84' may be less than the full width of the channel 76' to allow some flow to bypass the plate 84' (see alternative, reduced width, plate 84' shown in Fig. 5 in dashed lines). For the device 74' (shown in Fig. 6A), the channel 76' can be a photo-bioreactor (PBR) tube, a pond channel or a sloped raceway, as described above. Fig. 6A shows that the plate 84' of device 74' is pivotably mounted on the channel 76'. As shown, the device 74' also includes an activator 90', such as a rotary actuator, that is coupled with the plate 84' for selectively rotating the plate 84' about a pivot axis 92'. Specifically, the plate 84' can be rotated very quickly from a first plate position (shown in dotted lines in Fig. 6A) to a second plate position (shown in solid lines in Fig. 6A) to create a pulsed flow in the channel 76'. Unlike the embodiment shown in Fig. 6, a height differential between algal culture 82' on opposing sides of the plate 84' is not necessarily required for the embodiment shown in Fig. 6A. Instead, the activator 90' can be activated to rotate the plate 84' very quickly (see arrow 94') to pinch algal culture 82' between the plate 84' and channel floor 80' to generate a pulsed flow (illustrated as wave 96') and can generate turbulence (illustrated by eddy flow arrow 98') in the channel 76'. Fig. 6A also shows that the activator 90' can include controller 100' and a timer 102' for initiating a pulsed flow in accordance with a predetermined schedule during a pulse generation sequence (e.g. between time intervals of 2 minutes to six hours).

Fig. 6B shows an embodiment of a device 74" for generating a pulsed flow in a channel 76" having a channel floor 80" and containing a circulating algal culture 82". As shown, the device 74" includes a plate 84" that is positioned in the channel 76". The plate 84" may be less than the full width of the channel 76" to allow some flow to bypass the plate 84". For the device 74", the channel 76" can be a photo-bioreactor (PBR) tube, a pond channel or a sloped raceway, as described above. Fig. 6B shows that the plate 84" is pivotably mounted on the channel 76". The device 74" also includes an activator 90", such as a rotary actuator, that is coupled with the plate 84" for selectively rotating the plate 84" about a pivot axis 92". Specifically, the plate 84" can be rotated very quickly from a first plate position (shown in dotted lines in Fig. 6B) to a second, vertical, plate position (shown in solid lines in Fig. 6B) to create a pulsed flow in the channel 76". Unlike the embodiment shown in Fig. 6, a height differential between algal culture 82" on opposing sides of the plate 84" is not necessarily required for the embodiment shown in Fig. 6B. Instead, the activator 90" can be activated to rotate the plate 84" very quickly (see arrow 94") to push a pulse of algal culture 82" and generate a pulsed flow (illustrated as wave 96") and can generate turbulence (illustrated by eddy flow arrow 98") in the channel 76". Fig. 6B also shows that the activator 90" can include a controller 100" and a timer 102" for initiating a pulsed flow in accordance with a predetermined schedule during a pulse generation sequence (e.g. between time intervals of 2 minutes to six hours).

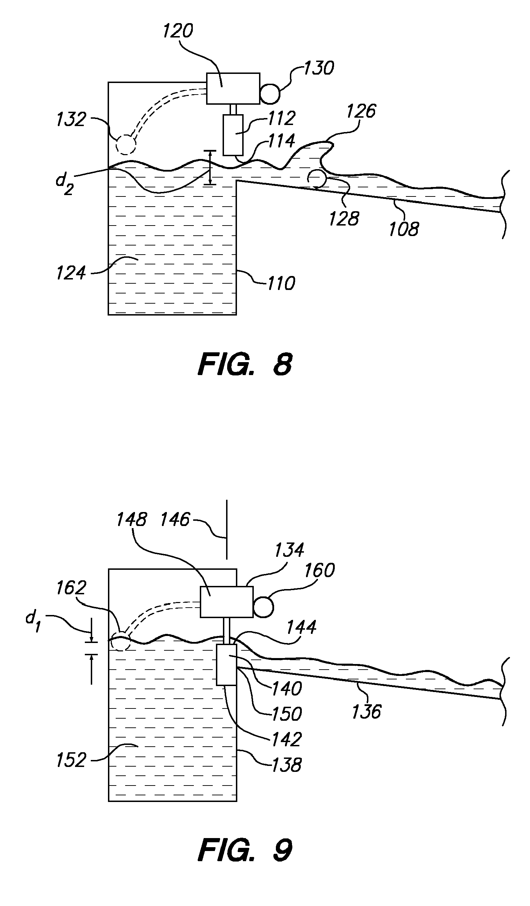

Figs. 7 and 8 show a device 106 for generating a pulsed flow in a sloped raceway 108 that is in fluid communication with a sump 110 can include a gate 112 that is formed as a planar plate. As shown, the gate 112 has a first end 114 and a second end 116 and defines a plane (extending into the plane of the page and containing axis 118. The gate 112 is interposed between the sump 110 and the sloped raceway 108 and is mounted on the sump-raceway assembly to allow a back and forth movement of the gate 112 within the defined plane. To control movement of the gate 112, the gate 112 is coupled to an activator 120, such as a linear actuator having an actuator rod. With this cooperative structural interaction, the gate 112 can be selectively moved within the defined plane from a first position to a second position to establish a so-called "pinch gate."

Continuing with reference to Figs. 7 and 8, it can be seen that the sump 110 is attached to the raceway 108 at an edge 122 that is submerged in algal culture 124. To create a pulse, the gate 112 is initially positioned to locate the first gate end 114 at a distance *d₁* from the submerged edge 122, allowing algal culture 124 to accumulate behind the gate 112 in the sump 110, as shown in Fig. 7. When a pulse is needed, the gate 112 is opened by moving it to the second gate position (shown in Fig. 8) where the first gate end 114 is at a distance *d₂* from the submerged edge 122, with d₂ > d₁. Note, for this embodiment, the gate 112 can be slightly open in the first position (d₁ > 0) to allow the algal culture 124 to circulate at some established minimum flow, or the gate 112 can be temporarily closed (d₁ = 0).

In the gate position shown in Fig. 7, a flow rate *f₁* is established from the sump 110 to the raceway 108 and in the gate position shown in Fig. 8 a flow rate *f₂* is established from the sump 110 to the raceway 108, with f₂ > f₁. To generate a pulsed flow in the raceway 108, the gate 112 is moved from the position shown in Fig. 7 to the position shown in Fig. 8. This release causes a pulsed flow (illustrated as wave 126 in Fig. 8) and can generate turbulence (illustrated by eddy flow arrow 128) in the algal culture 124 on the raceway 108. As shown, the activator 120 can include a timer 130 for initiating plate movements to establish pulsed flows in accordance with a predetermined schedule. Alternatively, or in addition to the timer 130, the activator 120 can include a level switch 132 (shown in dotted lines as optional) for measuring a height of algal culture 124 in the sump 110 and for initiating plate movements when the algal culture 124 reaches a predetermined height.

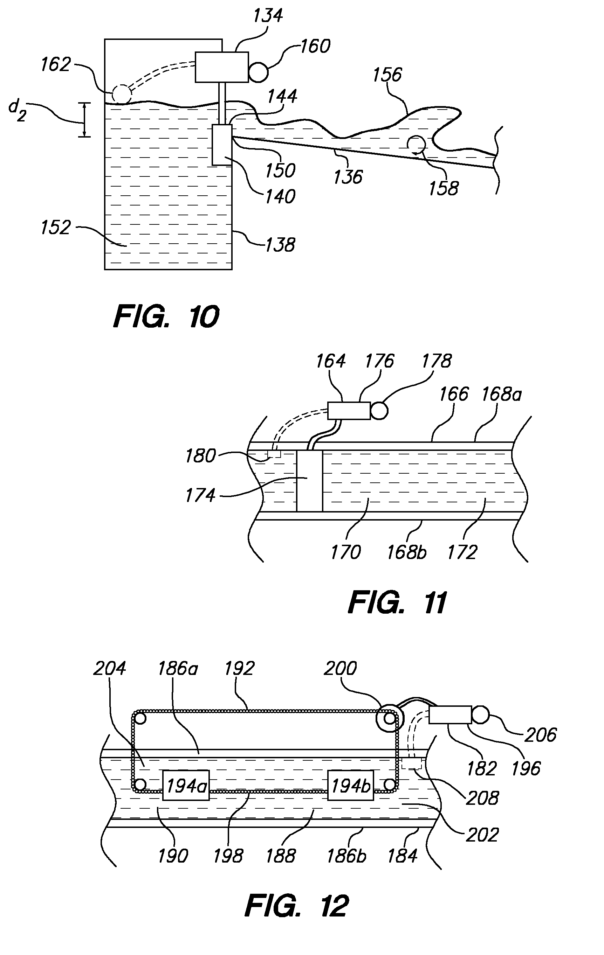

Figs. 9 and 10 show another embodiment of a device 134 for generating a pulsed flow in a sloped raceway 136 that is in fluid communication with a sump 138 can include a gate 140 that is formed as a planar plate. As shown, the gate 140 has a first end 142 and a second end 144 and defines a plane (extending into the plane of the page and containing axis 146. The gate 140 is interposed between the sump 138 and the sloped raceway 136 and is mounted on the sump-raceway assembly to allow a back and forth movement of the gate 140 within the defined plane. To control movement of the gate 140, the gate 140 is coupled to an activator 148, such as a linear actuator having an actuator rod. With this cooperative structural interaction, the gate 140 can be selectively moved within the defined plane from a first position to a second position to establish a so-called "overflow gate."

Continuing with reference to Figs. 9 and 10, it can be seen that the sump 138 is attached to the raceway 136 at an edge 150 that is submerged in algal culture 152. To create a pulse, the gate 140 is initially positioned to locate the second gate end 144 at a distance *D₁* below the surface of the algal culture 152 allowing algal culture 152 to accumulate behind the gate 140 in the sump 138, as shown in Fig. 9. When a pulse is needed, the gate 140 is opened by moving it to the second gate position (shown in Fig. 10) where the second gate end 144 is submerged at a distance *D₂* below the surface of the algal culture 152, with D₂ > D₁. Note, for this embodiment, the gate 140 can be slightly open in the first position D₁ > 0 (as shown in Fig. 9) to allow the algal culture 152 to circulate at some established minimum flow, or the gate 140 can be temporarily closed D₁ = 0 (not shown). In the gate position shown in Fig. 9, a flow rate *f₁* is established from the sump 138 to the raceway 136 and in the gate position shown in Fig. 10 a flow rate *f₂* is established from the sump 138 to the raceway 136, with f₂ > f₁. To generate a pulsed flow in the raceway 136, the gate 140 is moved from the position shown in Fig. 9 to the position shown in Fig. 10. This release causes a pulsed flow (illustrated as wave 156 in Fig. 10) and can generate turbulence (illustrated by eddy flow arrow 158) in the algal culture 152 on the raceway 136. As shown, the activator 148 can include a timer 160 for initiating plate movements to establish pulsed flows in accordance with a predetermined schedule. Alternatively, or in addition to the timer 160, the activator 148 can include a level switch 162 (shown in dotted lines as optional) for measuring a height of algal culture 152 in the sump 138 and for initiating plate movements when the algal culture 152 reaches a predetermined height.

Fig. 11 shows a device 164 for generating a pulsed flow in a channel 166 having channel walls 168a,b and a channel floor 170 and containing a circulating algal culture 172. As shown, the device 164 includes a pump 174 that is positioned in the channel 166. For the device 164, the channel 166 can be a photo-bioreactor (PBR) tube, a pond channel or a sloped raceway, as described above. As shown, the pump 174 is coupled to an activator 176 for selectively increasing the flow rate in the channel 166 from a first flow rate *f₁* established for circulating the algal culture 172 through the channel 166 to a second flow rate *f₂* with f₂ > f₁ to generate a pulsed flow in the channel 166. For example, the pump 174 can be a variable rate pump such as a centrifugal pump, a screw pump, an airlift pump, a propeller pump or a jet pump. In one setup, flow is increased to establish a pulsed flow by increasing the flow rate such that the ratio of flow rates (f₂ : f₁) is in a range between about 2:1 and about 10:1. As shown, the activator 176 can include a timer 178 for increasing pump flow to establish pulsed flows in accordance with a predetermined schedule. After the desired duration of the increased flow rate *f₂* has elapsed, the flow rate will decrease back to its flow rate *f₁*. Alternatively, or in addition to the timer 178, the activator 176 can include a level switch 180 (shown in dotted lines as optional) for measuring a height of algal culture 172 in the channel 166 and for increasing pump flow when the algal culture 172 reaches a predetermined height.

Fig. 12 shows an embodiment of a device 182 for generating a pulsed flow in a channel 184 having channel walls 186a,b and a channel floor 188 and containing a circulating algal culture 190. As shown, the device 182 includes a pump 192 having lifting buckets 194a,b and an activator 196 that includes a moving track 198 having a motor 200. The activator 196 is configured to selectively move the buckets 194a,b from a channel fill location 202 where a bucket 194a,b is filled to a channel dump location 204 where a bucket 194a,b is emptied. For this aspect, the activator 196 can include a timer 206 for initiating pump rate changes in accordance with a predetermined schedule and/or an optional level switch 208 for controlling pump operation. In some cases, the lifting buckets (not shown) can be configured to travel up and down, collecting fluid at a low point in the system and dumping the fluid at a high point. Between the low and high points are the flow channels for algae to grow. This method can be used for both open ponds and for tubular PBRs.

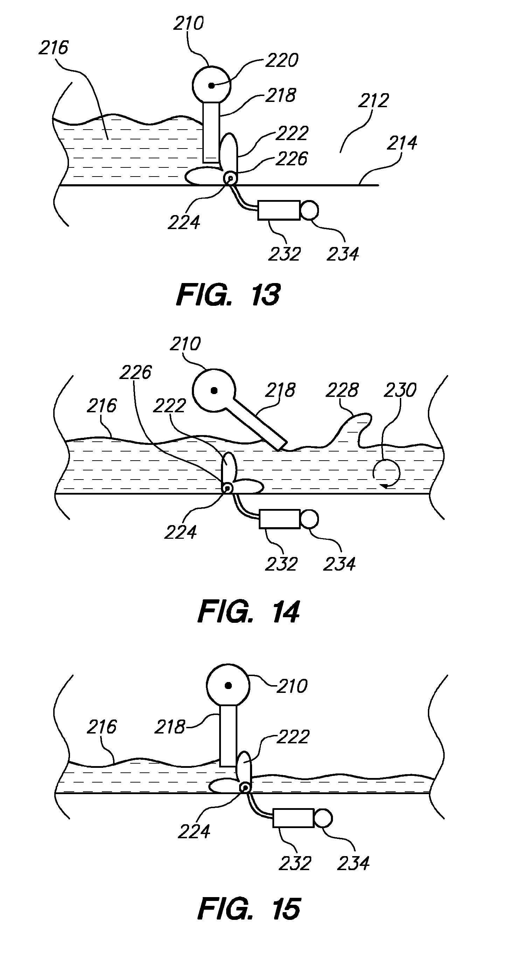

Figs. 13-15 show a device 210 for generating a pulsed flow in a channel 212 having channel floor 214 and containing a circulating algal culture 216. As shown, the device 210 includes a plate 218 that is positioned in the channel 212. For the device 210, the channel 212 can be a photo-bioreactor (PBR) tube, a pond channel or a sloped raceway, as described above. Figs. 13-15 illustrate that the plate 218 is pivotably mounted for rotation (e.g. free rotation) about a pivot axis 220. The device 210 also includes a holder/trigger (trigger 222) that is pivotably mounted on the channel 212 for rotation about a pivot axis 224 to selectively hold or release the plate 218. The plate 218 and/or the trigger 222 may be less than the full width of the channel 212 to allow some flow to bypass the plate 218/trigger 222 assembly when the plate 218 is in the closed position (closed position shown in Fig. 13 and Fig. 15). An activator 226, such as a rotary actuator, is coupled with the trigger 222 for selectively rotating the trigger 222 about the pivot axis 224. Specifically, the plate 218 can be released for free rotation in the flow of algal culture 216 from a first, closed plate position (shown in Fig. 13) to a second, open plate position (shown in Fig. 14). Fig. 13 also shows that the activator 226 can include controller 232 and a timer 234 for initiating a pulsed flow in accordance with a predetermined schedule.

In operation, a pulsed flow can be generated in the channel 212 by first holding the plate 218 in the closed position (Fig. 13) using the trigger 222. As shown in Fig. 13, in the first, closed position, the plate 218 impedes the flow of circulating algal culture 216 producing a height differential between algal culture 216 on opposing sides of the plate 218. After a preselected time, or when a preselected height differential across the plate 218 is achieved, the activator 226 can be activated to rotate the trigger 222 and release the plate 218 (see Fig. 14) to allow the buildup of algal culture 216 behind the plate 218 to flow, substantially unimpeded, along the channel 212. This release causes a pulsed flow (illustrated as wave 228) and can generate turbulence (illustrated by eddy flow arrow 230) in the channel 212. As shown in Fig. 15, once the buildup of algal culture 216 has passed the open plate 218, the plate 218 is free to rotate back to the closed position, due to gravity, where the trigger 222 can be rotated to hold the plate 218 in the closed position. The cycle shown in Fig. 13 to Fig. 15 can then be repeated to generate another pulsed flow.

While the System and Method for Using a Pulse Flow Circulation for Algae Cultivation as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as described in the appended claims.

| | | | |
|---|---|---|---|
| 10 | system #1 | 66 | flush gates (a-f) |
| 12 | photobioreactor (PBR) | 68 | segment (a-e) |
| 14 | inlet | 70 | light zone |
| 16 | outlet | 72 | dark zone |
| 18 | flow channel (a-h) | 74 | device (74' / 74") |
| 20 | transfer pipe | 76 | channel (76' / 76") |
| 22 | circulation pump | 78 | walls (a, b) |
| 24 | conduit | 80 | floor (80' / 80") |
| 26 | diverting valve | 82 | algae culture (82' / 82") |
| 28 | flush tank | 84 | plate (84' / 84") |
| 30 | inlet flush tank | 86 | end |
| 32 | outlet flush tank | 88 | end |
| 33 | activator | 90 | activator (90' / 90") |
| 34 | level switch | 92 | axis (92' / 92") |
| 36 | timer | 94 | arrow (94' / 94") |
| 38 | signal wire | 96 | wave (96' / 96") |
| 40 | gate valve | 98 | arrow (98' / 98") |
| 42 | transfer pipe | 100 | controller (100' / 100") |
| 44 | gas exchange tank | 102 | timer (102' / 102") |
| 46 | return pipe | 104 | level switch |
| 48 | system #2 | 106 | device |
| 50 | raceway pond | 108 | raceway |
| 52 | drainage point | 110 | sump |
| 54 | inlet | 112 | gate |
| 56 | flow arrows (a-b) | 114 | end |
| 58 | system #3 | 116 | end |
| 60 | sloped pond | 118 | axis |
| 62 | sump (a-b) | 120 | activator |
| 64 | main pump (a-b) | 122 | edge |
| 124 | algal culture | 184 | channel |
| 126 | wave | 186 | walls (a, b) |
| 128 | arrow | 188 | floor |
| 130 | timer | 190 | algae culture |
| 132 | level switch | 192 | pump |
| 134 | device | 194 | bucket (a, b) |
| 136 | raceway | 196 | activator |
| 138 | sump | 198 | track |
| 140 | gate | 200 | motor |
| 142 | end | 202 | location |
| 144 | end | 204 | location |
| 146 | axis | 206 | timer |
| 148 | activator | 208 | level switch |
| 150 | edge | 210 | device |
| 152 | algae culture | 212 | channel |
| 156 | wave | 214 | floor |
| 158 | arrow | 216 | culture |
| 160 | timer | 218 | plate |
| 162 | level switch | 220 | axis |
| 164 | device | 222 | trigger |
| 166 | channel | 224 | axis |
| 168 | walls (a, b) | 226 | activator |
| 170 | floor | 228 | wave |
| 172 | algae culture | 230 | arrow |
| 174 | pump | 232 | controller |
| 176 | activator | 234 | timer |
| 178 | timer | | |
| 180 | level switch | | |
| 182 | device | | |

## Claims

1. A device for generating a pulsed flow in a channel containing a circulating algal culture, the device comprising:
a plate positioned in the channel and having a first end and a second end with the first end pivotably mounted on the channel; and
an activator coupled with the plate for selectively rotating the plate about a pivot axis from a first position to a second position to generate a pulsed flow in a channel.

2. A device as recited in claim 1 wherein the activator comprises a rotary actuator.

3. A device as recited in claim 1 or 2 wherein the activator comprises a timer for initiating plate rotations in accordance with a predetermined schedule.

4. A device as recited in any preceding claim wherein the second end of the plate is positioned above the algal culture for a preselected period of time to establish a preselected height differential between algal culture on opposing sides of the plate.

5. A device as recited in any preceding claim wherein the second end of the plate is positioned above the algal culture in the first position and the second end of the plate is submerged in the algal culture in the second position.

6. A device as recited in any preceding claim wherein the channel is formed with a bottom and wherein the plate is rotated toward the channel bottom to generate a pulsed flow by pinching algal culture between the plate and the channel bottom.

7. A device as recited in claims 1 to 5 wherein the channel is formed with a bottom and wherein the plate is rotated away from the channel bottom to generate a pulsed flow by pushing algal culture between the plate and the channel bottom.

8. A device for generating a pulsed flow in a conduit holding a circulating algal culture, the conduit including a sump in fluid communication with a sloped raceway, the device comprising:
a gate formed as a planar plate defining a plane and having a first end and a second end, the gate being interposed between the sump and the sloped raceway and mounted for movement within the plane; and
an activator coupled to the plate for selectively moving the gate within the plane from a first position wherein a flow rate *f₁* is established from the sump to the raceway to a second position wherein a flow rate *f₂* is established from the sump to the raceway, with f₂ > f₁ to generate a pulsed flow in the conduit.

9. A device as recited in claim 8 wherein the gate has a first end and a second end and the sump is attached to the raceway at a submerged edge, and wherein the first end is located at a distance *d₁* from the submerged edge with the gate in the first position, and the first end is located at a distance *d₂* from the submerged edge with the gate in the second position, with d₂ > d₁.

10. A device as recited in claim 8 wherein the gate has a first end and a second end and wherein the second end of the gate is positioned above the algal culture in the first position and the second end of the gate is submerged in the algal culture in the second position.

11. A device for generating a pulsed flow in a channel containing a circulating algal culture, the device comprising:
a pump positioned for interacting with the algal culture; and
an activator coupled to the pump for selectively operating the pump to increase the flow rate in the channel from a first flow rate *f₁*, established for circulating the algal culture through the channel, to a second flow rate *f₂* with f₂ > f₁ to generate a pulsed flow in the channel.

12. A device as recited in claim 11 wherein the pump is a variable rate pump.

13. A device as recited in claim 11 or 12 wherein the pump is selected from the group of pumps consisting of a centrifugal pump, a screw pump, a propeller pump, a jet pump and an airlift pump.

14. A device for generating a pulsed flow in a channel containing a circulating algal culture, the device comprising:
at least one lifting bucket positioned for interacting with the algal culture; and
an actuator having a motorized track for moving the bucket from a channel fill location to a channel dump location to selectively increase the flow rate in the channel from a first flow rate *f₁*, established for circulating the algal culture through the channel, to a second flow rate *f₂* with f₂ > f₁ to generate a pulsed flow in the channel.

15. A device for generating a pulsed flow in a channel containing a circulating algal culture to increase exposed algal surface area and improve productivity, the device comprising:
a plate positioned in the channel and having a first end and a second end with the first end pivotably mounted;
a trigger rotatable about a pivot point; and
an activator coupled with the trigger for selectively rotating the trigger from a first position wherein the trigger holds the plate in a closed position to a second position in which the plate is released into an open position to generate a pulsed flow in a channel.
